# EUROPEAN PATENT APPLICATION

(11) **EP 4 736 792 A1**
(43) Date of publication of application: **06.05.2026**
(21) Application number: 24940784.2
(22) Date of filing: 07.05.2024
(51) Int. Cl.: A61B 17/34, B01D 46/42, B01D 53/26

(54) **FILTER MODULE HAVING FLOW MARKER FUNCTION OR MOISTURE REMOVAL FUNCTION**

(71) Applicant: Kim, Hyun-Young, Bucheon-si, Gyeonggi-do 14669 (KR)
(72) Inventor: Kim, Hyun-Young, Bucheon-si, Gyeonggi-do 14669 (KR)
(74) Representative: Banse & Steglich Patentanwälte PartmbB
(86) International application number: PCT/KR2024/006108
(87) International publication number: WO 2025/234507

(57) **Abstract**

The present invention relates to a filter module with a flow indicating function or a humidity removing function. A filter module with a flowing indicator comprises a flow indicating module 11 capable of identifying whether or not a fluid flows through; a flowing tube 12 connected to one part of the flow indicating module 11 and guiding a gas containing a bacterium or a germ discharged from a surgery tool to the flow indicating module 11; and a filter unit 13 connected to the other part of the flow indicating module 11.

## Description

### BACKGROUND OF THE INVENTION

### 1. Field of the Invention

The present invention relates to a filter module with a flow indicating function or a moisture removing function, in particular the filter module with a flowing indicator for checking a gas to flow to the filter or with the moisture removing function to remove the moisture from the gas flowing from an inside of a human body to the filter.

### Description of the Related Art

A gas may be injected to an inside of a human body through a trocar or a surgical tool similar to the trocar used for a surgery such as a laparoscopic surgery, and a gas including a virus, a bacterium, a germ or a smoke generated within the human body may be discharged to an outside of the human body through a predetermined passage. The gas discharged to the outside contains a smoke as well as various odor components generated during a surgery, and therefore the gas has to be discharged through a filter. Aa the gas is discharged very slowly through the filter in the process of being discharged from the trocar to the outside via a flowing tube and the smoke filter, it is difficult to be verified whether or not the gas is being actually discharged. Therefore, it is necessary for a means for checking a discharge of a gas or a flow of a gas to be made, but the known skills do not disclose such means.

And also, a gas generated during a surgery of a human body may contain a smoke component, an odor component or the like, and the gas may contain a moisture. The smoke component and the odor component may be removed by a filter, but the moisture may not be removed because there is no further removing means. If the moisture flows and is absorbed by the filter, a performance of the filter may be reduced or a function of the filter may be lost. And a surgery tool or a medical personnel in a surgery room may be infected with a bacterium, a virus or the like after the function of the filter is lost. Furthermore, there is a problem that the filter has to be replaced more frequently. Therefore, the moisture contained at the gas need to be removed before reaching the filter, but the prior art does not disclose such skills.

The present invention is intended to solve the problems of the prior art and have the following purposes.

### PURPOSE OF THE INVENTION

One object of the present invention is to provide with a filter module with a flow indicator to verify a gas containing a virus, a bacterium or a germ generated within a human body to flow to a filter through a flowing tube.

Another object of the present invention is to provide with a filter module with a function for removing a moisture within a flowing gas during flowing to a filter, wherein the filter module is capable of removing the moisture within the gas generated in the course of a surgery.

### SUMMARY OF THE INVENTION

In one embodiment of the present invention, a filter module with a flowing indicator comprises a flow indicating module capable of identifying whether or not a fluid flows through; a flowing tube connected to one part of the flow indicating module and guiding a gas containing a bacterium or a germ discharged from a surgery tool to the flow indicating module; and a filter unit 13 connected to the other part of the flow indicating module.

In other embodiment of the present invention, the flow indicating module comprises a rotating wing unit rotated by a fluid pressure or a flowing ball.

In another embodiment of the present invention, the flow indicating module comprises at least one rotating ball disposed with a flowing cylinder or a guiding flap formed within a flowing valve and having a flexible property.

In still another embodiment of the present invention, a filter module capable of removing a moisture comprises a discharging means for flowing a gas component generated during a surgery through a first flowing tube; a moisture removing module for removing the moisture from the gas component flowing through the first flowing tube; and a filter unit for purifying and discharging the gas component flowing through a second flowing tube in a state that the moisture is removed.

In still another embodiment of the present invention, the filter module further comprises a selective filter formed at a passage of the first flowing tube or the second flowing tube.

In still another embodiment of the present invention, the moisture removing module comprises an enclosed area, and a moisture absorbing unit 82 is disposed within the moisture removing module.

In still another embodiment of the present invention, the first flowing tube or the second flowing tube further comprises at least one tilted passage.

### BRIEF DESCRIPTION OF THE DRAWINGS

FIG. 1 shows an embodiment of a filter module with a flowing indicator according to the present invention.
FIG. 2 shows one embodiment of the flow indicating module for the filter module according to the present invention.
FIG. 3 shows another embodiment of the flow indicating module for the filer module according the present invention.
FIG. 4 shows still another embodiment of the flow indicating module for the filter module according to the present invention.
FIG. 5 shows still another embodiment of the flow indicating module for the filter module according to the present invention.
FIG. 6 shows an embodiment of a surgery tool to which the filter module is applied according to the present invention.
FIG. 7 shows an embodiment of a filter module capable of removing a moisture according to the present invention.
FIG. 8 shows an embodiment of a surgery tool to which the filter module capable of removing a moisture is applied according to the present invention.
FIG. 9, 10 and 11 show embodiments of various surgery tools to which the filter module capable of removing a moisture is applied according to the present invention.

### DETAILED DESCRIPTION OF EXEMPLARY EMBODIMENTS

Exemplary embodiments of the present invention will be described herein below with reference to the accompanying drawings.

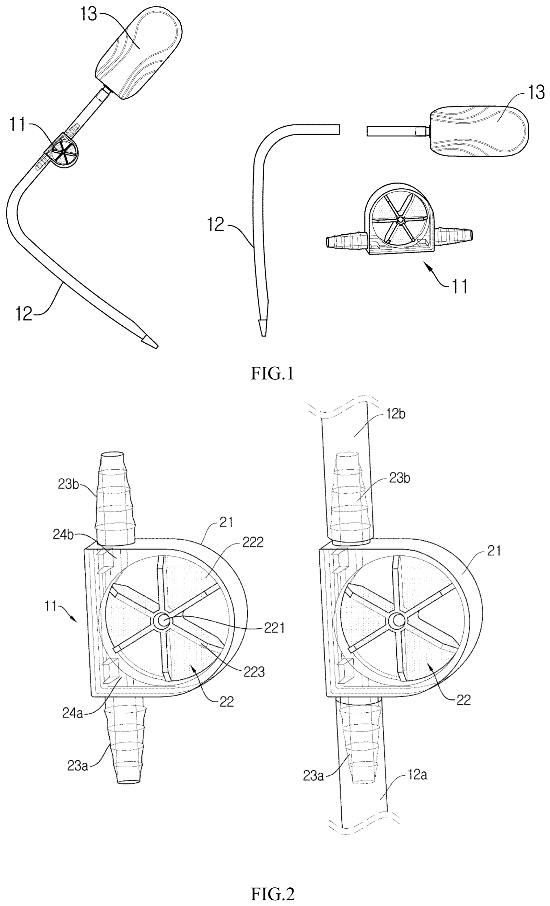

FIG. 1 shows an embodiment of a filter module with a flowing indicator according to the present invention.

Referring to FIG. 1, a filter module with a flowing indicator comprises a flow indicating module 11 capable of identifying whether or not a fluid flows through; a flowing tube 12 connected to one part of the flow indicating module 11 and guiding a gas containing a bacterium or a germ discharged from a surgery tool to the flow indicating module 11; and a filter unit 13 connected to the other part of the flow indicating module 11.

The flow indicating module 11 may form a part of a flowing passage of the gas containing a virus, a bacterium or a germ, and the flow indicating module 11 may allow a flow of gas to be verified visually. The flow indicating module 11 may comprise a flowing indicator which moves, changes a shape or changes a color by a gas flow. The flowing indicator may have an identifiable shape, and preferably have a shape identified with a naked eye. The flowing tube 12 may be connected to one part of the flow indicating module 11, and the flowing tube 12 may be connected to a surgery tool. The gas component containing a smoke, an odor, a virus, a bacterium or a germ generated from an inside of a human body may be introduced to the flowing tube 12 through an outlet formed at the surgery tool during a surgery. The gas may flow through the flowing tube 12 to enter the flow indicating module 11. The flowing indicator may indicate a flow state of the gas, and the gas may be guided to the filter unit 13 via the flow indicating module 11. The filter unit 13 may discharge a purified gas to an outside, after the moisture, the smoke, the odor component, the virus, the bacterium or the germ are removed from the gas.

If the gas inputs into the filter unit 13, a deteriorated gas may be filtered at first by a pre-filer and an absorbing filter for absorbing the moisture. And then the gas may pass through carbon layers equipped above and below and the smoke or the odor component contained in the gas may be removed. And then the gas from which the smoke or the odor component is removed in this way may pass through a functional filter disposed at the outer layer above or below, and the virus, the bacterium or the germ may be removed. And the purified gas may be discharged to the outside of the filter unit 13 through three filtering processes. The flow indicating module 11 may have a function indicating whether or not the gas flows through, and the flow indicating module 11 may have various structures identified with a naked eye.

FIG. 2 shows one embodiment of the flow indicating module for the filter module according to the present invention.

Referring to FIG. 2, the flow indicating module 11 comprises an indicating case 21; a rotating wing unit 22 rotating within the indicating case 21; an inlet 23a formed at one part of the indicating case 21; and an outlet 23b formed at the other part of the indicating case 21. The inlet 23a and the outlet 23b may be coupled in a way to be inserted to a first flowing tube 12a and a second flowing tube 12b, respectively, and the flow indicating module 11 may form a part the gas flowing passage. The inlet 23a may be connected to a first guiding passage 24a, and the first guiding passage 24a may be connected to an inside of the indicating case 21 having an enclosed structure. The outlet 23b may have a configuration similar to that of the inlet 23a.

The indicating case 21 may have an overall cylindrical shape, and the rotating wing unit 22 may be disposed within the indicating case 21 in a rotatable way. The rotating wing unit 21 may comprise a rotating center 221 coupled to a center of the indicating case 21; at least one or more rotating wing 222 extending to a radial direction from the rotating center 221 in a sector shape; a pressuring wall 223 formed along an edge of the rotating wing 222. The gas introduced to the first guiding passage 24a may pressurize the pressing wall 223, thereby the rotating wing 222 may be rotated. For example, three rotating wings 222 having a circumferential angel of 15 to 45 degree angle may be formed, and the gas introduced to a volume formed by the rotating wings 222 being adjacent each other may pressurize the pressing wall 223 to rotate the rotating wings 222. As the rotating wings 222 rotates, the gas may flow to the second guiding passage 24b, and the second guiding passage 24b may be connected to the outlet 23b. In such a way, the gas introduced to the inlet 23a through the first flowing tube 12a may enter an inside of the indicating case 21 through the first guiding passage 24a to rotate the rotating wings 222, and may be discharged to the outlet 23b through the second guiding passage 24b. And then, the gas may be guided to the filter unit through the outlet 24b and via the second flowing tube 12b. The flowing indicating module 11 may have various structures, but not limited to.

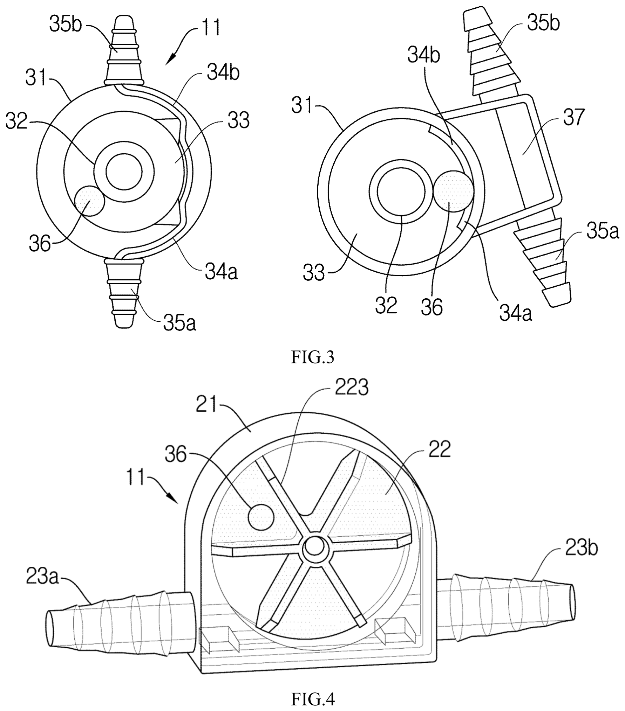

FIG. 3 shows another embodiment of the flow indicating module for the filer module according the present invention.

Referring to FIG.3, the flowing indicator may become one or more flowing ball 36. The flow indicating module 11 may comprise a circular case 31; a center guiding part 32 formed at a center of the circular case 31; an indicating passage 33 formed in a circular shape between an inner wall of the circular case 31 and the center guiding part 32; a first guiding passage 34a connected to one part of the indicating passage 33; a first guiding passage 34b connected to the other part of the indicating passage 33; an inlet 35a connected to the first guiding passage 34a; an outlet 35b connected to the second guiding passage 34b; and a flowing ball 36 disposed along the indicating passage 33 in a rotatable way. The circular case 31 may have a cylindrical block shape extending in a circular shape, and a flowing volume may be formed within the circular case 31 in a cylindrical shape. The center guiding part 32 of the cylindrical block shape may be formed at a center of the flowing volume, and the indicating passage 33 may be formed by double circular walls extending in a separated way each other. A width of the indicating passage 33 may correspond to a diameter of the flowing ball 36 with a spherical shape, and the flowing ball 36 may have a hollow spherical bead shape movable easily by a small pressure.

As shown in the left side of FIG. 3, the inlet 35a and the outlet 35b may extend in a shape to bisect the circular case 31. And also, referring to the right side of FIG. 3, a flow connecting block 37 may be formed at one part of the circular case 31, and the inlet 35a and the outlet 35b may be connected to the flow connecting block 37. A connecting passage connected to the inlet 35a and the outlet 35b may be formed within the flow connecting block 37, and the connecting passage may have function equal or similar to that of the first and second guiding passage 34a, 34b. In such structure, the gas flowing along the flowing tube may be introduced to the flow indicating module 11 through the inlet 35a. And the gas that enters through the inlet 35a may be guided to the indicating passage 33 through the first guiding passage 34a, and may rotate the flowing ball 36 disposed at the indicating passage 33. After the gas flows along the indicating passage 33 with the gas rotating the flowing ball 36, the gas may flow to the second guiding passage 34b to move to the outlet 35b. And then, the gas may flow to the flowing tube to be guided to the filter unit. Accordingly, the flow of the gas may be verified by the rotation of the flowing ball 36, and the flowing ball 36 may be made in color or structure that are easily visible, but not limited to.

FIG. 4 shows still another embodiment of the flow indicating module for the filter module according to the present invention.

Referring to FIG. 4, the rotating wing unit 22 having three wings may be disposed within the indicating case 21, and the flowing ball 36 may be displaced within an inner volume formed by pressing walls 223 of one rotating wing unit 22. The rotating wing unit 22 may rotate by the gas introduced through the inlet 23a, and at the same time, the flowing ball 36 may rotate. In this way, the rotating wing unit 22 and the flowing ball 36 may rotate together to verify a flow of the gas easily. The flow indicating module 11 may be made in various structures, but not limited to.

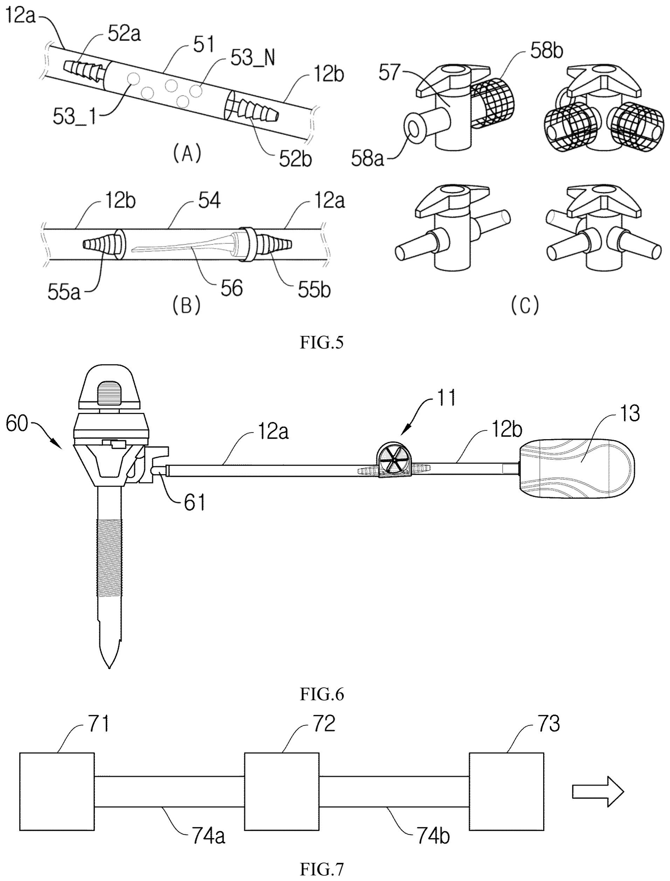

FIG. 5 shows still another embodiment of the flow indicating module for the filter module according to the present invention.

Referring to (A) of FIG. 5, the flow indicating module 11 may comprise at least one rotating balls 53_1 to 53_N disposed within a flowing cylinder 51. Inlets 52a, 52b connected to the first and second flowing tube 12a, 12b, respectively, may be formed at one end and the other end of the flowing cylinder 51. At least one rotating balls 53_1 to 53_N made from a synthetic resin form or a similar lightweight material and having a spherical shape or the like may be disposed within the flowing cylinder 51, and the rotating balls 53_1 to 53_N may have a color. The rotating balls 53_1 to 53_N may rotate by the gas introduced into the flowing cylinder 51 and flowing through the flowing cylinder 51, and thereby, it may be visually confirmed that the gas is flowing within the flowing cylinder 51. Selectively, the flowing cylinder 51 may have a function of a check valve, but not limited to.

Referring to (B) of FIG. 5, a guiding flap 56 may be disposed at an inside of a flowing valve 54, and an inlet 55a and an outlet 55b may be formed at one end and the other end of the flowing valve 54, and the inlet 55a and the outlet 55b may be connected to the first and the second flowing valve 12a, 12b, respectively. One end of the guiding flap 56 may be connected to the inlet 55a to extend along the inside of the cylindrical shape of the flowing valve 54 for guiding the gas. The guiding flap 56 may be made from a thin cloth, a flexible film, a flexible fabric or a similar material, and may be made from a material to be arranged along a flow direction of the gas. Selectively, the guiding flap 56 may be made from a material with a color. As the gas is introduced through the inlet 55a, the gas can flow into a front part of the guiding flap 56 connected to the inlet 55a. And as a result, the guiding flap 56 may be lined up parallel to the flowing valve 54. Thereby the gas flow can be verified visually. A portion of the guiding flap 56 which is connected to the inlet 55a may have a cylindrical shape. And at the same time, the portion may have a shape that two linear tails are overlapped each other along the extending direction, but not limited to.

Referring to (C) of FIG, 5, the first and the second flowing tubes 12a, 12b may be a first and a second connector 58a, 58b formed at a transparent check valve 57, respectively, and the flowing ball similar to those which is described above may be disposed within the check valve 57. As the gas flows along the inside of the check valve 57, the flowing ball can rotate and thereby the gas flow can be verified visually. The flow indicating module may be made in various structures, but not limited to.

FIG. 6 shows an embodiment of a surgery tool to which the filter module is applied according to the present invention.

Referring to FIG.6, the gas may be injected within a trocar 60 in the course of a surgery, and the gas containing a smoke, a contaminated material, a virus, a bacterium or a germ may be discharged to the outside of the trocar 60 through a discharging connector 61. One end of the first flowing tube 12a may be connected to the discharging connector 61, and the other end of the first tube 12a may be connected to one end of the flow indicating module 11. One end of the second flowing tube 12b may be connected to the other end of the flow indicating module 11, and the filter unit 13 may be connected to the other end of the second flowing tube 12b. The gas generated from an inside of a human body in the course of a surgery may flow upward along the trocar 60 to flow along the first tube 12a and along discharging connector 61. And then, the gas may enter the flow indicating module 11. As the gas flows along the inside of the flow indicating module 11, the gas can move the flow indicator, and thereby, the gas flow may be checked visually by an operator. The gas flowing from the flow indicating module 11 to the second flowing tube 12b may be purified at the filter unit 13 to be discharged to the outside. The flow indicating module 11 may be applied to various surgery tools to allow the flow state of the gas flow to be verified visually, but not limited to.

FIG. 7 shows an embodiment of a filter module capable of removing a moisture according to the present invention.

Referring to FIG. 7, a filer module having a function to remove a moisture from a flowing gas may comprise a discharging means 71 for making a gas component generated during a surgery flow through a first flowing tube 74a; a moisture removing module 72 for removing the moisture from the gas component flowing through the first flowing tube 74a; and a filter unit 73 for purifying and discharging the gas component flowing through a second flowing tube 74b in a state that the moisture is removed. The discharging means 71 may become various surgery tools to discharge various gases generated from a human body to the outside the human body, for example, the discharging means 71 may be a trocar used for a laparoscopic surgery. The gas generated from the inside of the human body may be introduced to the inside of the discharging means 71, and then the gas may be discharged to the outside through an outlet arranged at the discharging means 71. The outlet can be connected to the first flowing tube 74a in a way to allow the gas to flow, and the gas generated from the inside of the human body may flow along the first flowing tube 74a. The gas flowing along the first flowing tube 74a may pass through the moisture removing module 72, and the moisture contained in the gas may be removed from the moisture removing module 72. The moisture removing module 72 may comprise a stuff capable of absorbing the moisture, and the moisture removing module 72 may comprise, for example, a pulp, a SAP (super absorbent polymer) resin, a viscose rayon, a polypropylene, a PET (polyethylene terephthalate), a nylon, a cotton, a polyurethane, an acrylic, a cupro, a tencel, a wool, other miscellaneous cotton or a similar absorbent material. Such absorbent material may have a thickness of 1 to 30 mm, and may have a weight of 15 to 1,000 g/m(1m×1m), but not limited to. The gas from which the moisture is removed by the moisture removing module 72 may flow into the filter unit 73 through the second flowing tube 74b. The filter unit 73 may comprise at least one filter capable of removing the smoke, the odor, the bacterium or the virus component contained in the gas. The gas from which various kinds of foreign matters are removed passing through the filter unit 72 may be discharged to the outside in a purified condition. The filter unit 73 may comprise filters with various purposes, but not limited to.

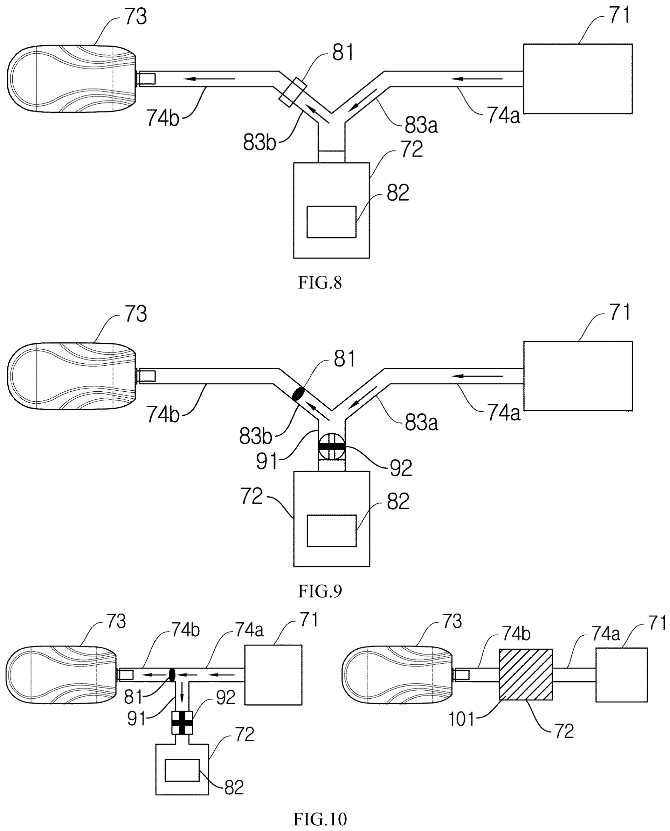

FIG. 8 shows an embodiment of a surgery tool to which the filter module capable of removing a moisture is applied according to the present invention.

Referring to FIG.8, the filter module may further comprise a selective filter 81 formed at the passage of the first flowing tube 74a or the second flowing tube 74b. And also, the moisture removing module 72 may form an enclosed area, and a moisture absorbing unit 82 may be disposed at an inside of the moisture removing module 72. In addition, the first flowing tube 74a or the second flowing tube 74b comprises at least one inclined passage 83a, 83b. The gas discharged from the discharging means 71 such as a surgery tool may be purified as it is introduced into the filter unit 73 through the first flowing tube 74a and the second flowing tube 74b. The moisture removing module 72 may be arranged between the first flowing tube 74a and the second flowing tube 74b, and the gas may flow through the moisture removing module 72.

The moisture removing module 72 may be made as an enclosed structure within which a receiving volume is formed, and the moisture removing module 72 may have a shape similar to that of a bottle or a vase. A connecting passage may be formed above the moisture removing module 72, and the connecting passage may be connected to each one end of the first and the second flowing tube 74a, 74b. For example, an upper end part of the connecting passage may have a shape of a pair of branches, and the first and the second flowing tube 74a, 74b may be connected to each branch, respectively. An end part of the first flowing tube 74a may have a bent shape, and the portion extending in a bent shape may form a first inclined passage 83a. An initiating portion of the second flowing tube 74b may be connected to the moisture removing module 72, and the second flowing tube 74b may comprise an inclined extending part connected to the moisture removing module 72. The inclined extending portion may form a second inclined passage 83b. At the second inclined passage 83b, the flowing tube 74b may extend in a bent shape from the end part of the second inclined passage 83b. In this way the first and the second inclined passage 83a, 83b may have a bent shape in a V-form, and the moisture removing module 72 may be connected to a lower vertex of the V-shape.

The selective filter 81 may be disposed at the first or the second inclined passage 83a or 83b, and the selective filter 81 may be made of a vapor-permeable water-proof fabric or a material having a similar vapor-permeable water-proof property. A liquid filtered by the selective filter 81 may flow through the first or the second inclined passage 83a or 83b to be collected at the moisture removing module 72. The moisture absorbing unit 82 may be disposed within the moisture removing module 72, and the moisture absorbing unit 82 may be made of, for example, a pulp, a viscose rayon, a polypropylene, a PET, a nylon, a cotton, a polyurethane, an acryl, a cupro, a tencel, a wool, other miscellaneous cotton or a similar material. And also, such absorbent material may have, for example, a thickness of 1 to 30 mm and a weight of 15 to 1,000 g/m (1m×1m), but not limited to. The first and the second flowing tube 74a, 74b or the moisture absorbent module 72 may be connected in various ways, and will be discussed in detail below

FIG. 9, 10 and 11 show embodiments of various surgery tools to which the filter module capable of removing a moisture is applied according to the present invention.

Referring to FIG. 9, a connecting passage 91 may be formed at the moisture absorbent module 72, and the first and the second flowing tube 74a, 74b may be connected to the connecting passage 91. The moisture absorbent unit 82 may be disposed within the moisture removing module 72, and the connecting passage 91 may protrude from the moisture removing module 72 to the outside, and a flowing passage may be formed within the connecting passage 91. A valve 92 may be disposed at the connecting passage 91, and the connecting passage 91 may be connected or may not be connected to an inside of the moisture removing module 72. When the valve 92 is in a closed state, the gas may flow from the first flowing tube 74a to the second flowing tube 74b via the first inclined passage 83a and the second inclined passage 83b. In contrast, when the valve 92 is in an open state, the gas can flow from the first inclined passage 83a into the moisture removing module 72 and next from the moisture removing module 72 to the second inclined passage 83b. Selectively, the moisture removing module 72 may be coupled to the connecting passage 91 in a removable or replaceable way. The moisture removing module 72 may have a function of removing the moisture by the moisture absorbing unit 82 disposed therein. And also, the moisture removing module 72 may have a function of collecting a moisture not passing through the selective filter 81 and flowing downward along the second inclined passage 83b. The moisture removing module 72 may have various functions, and the first and the second inclined passage 83a, 83b may be formed selectively.

Referring to the left part of FIG. 10, the moisture absorbent unit 82 may be disposed within the moisture removing module 72, and the connecting passage 91 may be formed at the moisture removing module 72. And also, the valve 92 may be disposed at the connecting passage 91 to connect the connecting passage 91 and an inside of the moisture removing module 72. The first flowing tube 74a may be connected to the second flowing tube 74b in a linear way, and the connecting tube 91 may be connected to the first flowing tube 74a or the second flowing tube 74 in a vertical way. A selective filter 81 may be disposed at a boundary portion of the second flowing tube 74b and the connecting passage 91, and the selective filter 81 may have a moisture absorbing function or have a function of preventing the moisture from passing through. The gas discharged through the discharging means 71 may flow along the first flowing tube 74a to be introduced into the moisture removing module 72 with the valve 92 opened. And after the moisture is removed at the moisture removing module 71 by the moisture absorbing material 82, the gas may flow to the second flowing tube 74b. Before the gas flows to the second flowing tube 74b, a residual moisture included in the gas may be removed by the selective filer 81. And the gas from which the residual moisture is removed may be discharged to the outside after being purified by the filter module 73. The moisture removed by the selective filter 81 may be collected at the moisture removing module 72. The moisture removing module 72 may have a replaceable structure, and for example, the valve 92 may be locked for replacing the moisture removing module 72. And then, after the moisture removing module 72 is replaced, the valve 92 may be opened in order that the gas may flow into the inside of the replaced moisture removing module 72.

Referring to the right part of FIG. 10, the moisture removing module 72 may be disposed at a route connecting the first flowing tube 74a and the second flowing tube in a linear way. The moisture removing module 72 may have an enclosed box shape, and the first and the second flowing tube 74a, 74b may be connected to both sides of the moisture removing module 72, respectively. A moisture absorbent agent 101 may be filled within the moisture removing module 72, and the moisture absorbent agent 101 may become, for example, a pulp material, a cotton, a sponge, a viscose rayon, SAP (Super Absorbent Polymer) resin, a polypropylene, a PET, a nylon, a polyurethane, an acryl, a cupro, a tencel, a wool, other miscellaneous cotton or a similar absorbent material. And also, the moisture absorbent agent 101 may become a porous material, for example, a porous zeolite, a porous non-woven fabric, a porous polyolefin or a similar porous absorbent material. Selectively, the moisture removing module 72 may be made in an openable structure, for example, one surface of the moisture removing module 72 may be made in an openable structure for replacing the moisture absorbent agent 101. The moisture removing module 72 may be made in various structures, but not limited to.

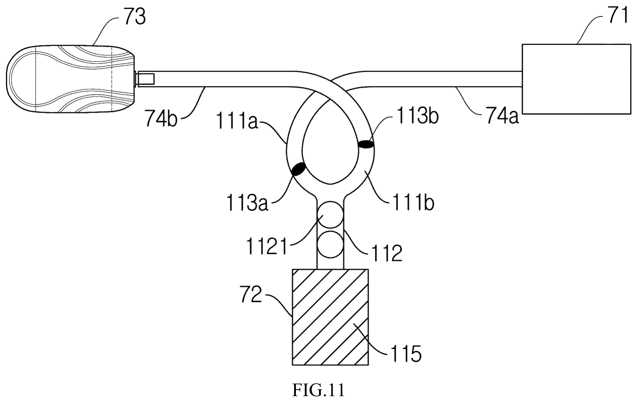

Referring to FIG. 11, a connecting passage 112 may be formed at the moisture removing module 72, and a moisture absorbent sheet 115 such as a SAP (Super Absorbent Polymer) sheet may be filled within the moisture removing module 72. A first curved flowing passage 111a may be formed at the first flowing tube 74a, and the second flowing tube 74b may be connected to a second curved flowing passage 111b. The first curved flowing passage 111a and the second curved flowing passage 111b may become a semi-circular shape, respectively, and a connecting passage 112 may be connected to an end part and an initiating part of the first and the second curved flowing passage 111a, 111b in an integrating way. Hereby, the first curved flowing passage 111a, the second curved flowing passage 111b and the connecting passage 112 may form three branch passages. The first and the second curved flowing passage 111a, 111b may have a circular shape to be connected to the first and the second flowing tube 74a, 74b, and thereby, an end part of the first flowing tube 74a and an initiating part of the second flowing tube 74b may have an overlapped structure each other. A removing filter 113a and an absorbing filter 113b may be disposed at the first and the second curved flowing passage 111a, 111b, respectively. The removing filter 111a may have a function of allowing the gas to pass through but the moisture not to pass through, and the absorbent filter 113b may be made of various materials capable of absorbing a moisture. The moisture removing module 72 may be coupled to the connecting passage 112 by a coupling portion 1121 in a removable way. The removing filter 113a or the absorbent filter 113b may be made of various materials, but not limited to.

While the present invention has been particularly shown and described with reference to exemplary embodiments thereof, it will be understood by those skilled in the art that various changes in form and details may be made therein without departing from the spirit and scope of the present invention as defined by the appended claims.

## Claims

1. A filter module with a flowing indicator comprising:
a flow indicating module (11) capable of identifying whether or not a fluid flows through;
a flowing tube (12) connected to one part of the flow indicating module (11) and guiding a gas containing a bacterium or a germ discharged from a surgery tool to the flow indicating module (11); and
a filter unit (13) connected to the other part of the flow indicating module (11).

2. The filter module according to claim 1, wherein the flow indicating module (11) comprises a rotating wing unit (22) rotated by a fluid pressure or a flowing ball (36).

3. The filter module according to claim 1, wherein the flow indicating module (11) comprises at least one rotating ball (53_1 to 53_N) disposed with a flowing cylinder (51) or a guiding flap (56) formed within a flowing valve (54) and having a flexible property.

4. A filter module capable of removing a moisture comprising:
a discharging means (71) for flowing a gas component generated during a surgery through a first flowing tube (74a);
a moisture removing module (72) for removing the moisture from the gas component flowing through the first flowing tube (74a); and
a filter unit (73) for purifying and discharging the gas component flowing through a second flowing tube (74b) in a state that the moisture is removed.

5. The filter module according to claim 1, wherein the filter module further comprises a selective filter (81) formed at a passage of the first flowing tube (74a) or the second flowing tube (74b).

6. The filter module according to claim 4, wherein the moisture removing module (72) comprises an enclosed area, and a moisture absorbing unit (82) is disposed within the moisture removing module (72).

7. The filter module according to claim 4, wherein the first flowing tube (74a) or the second flowing tube (74b) further comprises at least one tilted passage (83a, 83b).
